(19) Europäisches Patentamt
European Patent Office
Office européen des brevets

(11) Publication number: **0 160 079**
**B1**

(12) # EUROPEAN PATENT SPECIFICATION

(45) Date of publication of patent specification: **31.01.90**

(51) Int. Cl.5: **A 61 N 5/10**, A 61 K 31/70, C 07 H 17/00

(21) Application number: **84904049.8**

(22) Date of filing: **26.10.84**

(86) International application number: **PCT/US84/01735**

(87) International publication number: **WO 85/01871 09.05.85 Gazette 85/11**

(54) **METHOD AND MATERIALS FOR SENSITIZING NEOPLASTIC TISSUE TO RADIATION.**

(30) Priority: **26.10.83 US 545693**

(43) Date of publication of application: **06.11.85 Bulletin 85/45**

(45) Publication of the grant of the patent: **31.01.90 Bulletin 90/05**

(84) Designated Contracting States: **AT BE CH DE FR GB LI LU NL SE**

(56) References cited:
**DD-A- 147 774**
**US-A-4 017 606**
**US-A-4 210 638**
**JOURNAL OF MEDICINAL CHEMISTRY, vol. 22, no. 1, 1979, pages 21-24, American Chemical Society, Columbus, Ohio, US; K.A. WATANABE et al.: "Nucleosides. 110. Synthesis and antiherpes virus activity of some 2'-fluoro-2'-deoxyarabinofuranosylpyrimidine nucleosides"**
**CHEMICAL ABSTRACTS, vol. 86, 1977, page 440, abstract no. 16900e, Columbus, Ohio, US; & HU - A - 11 442 (GYOGYSZERKUTATO INTEZET) 28-04-1976**

(73) Proprietor: **GREER, Sheldon B.**
**8320 S W 86 Terrace**
**Miami, FL 33143 (US)**

(72) Inventor: **GREER, Sheldon B.**
**8320 S W 86 Terrace**
**Miami, FL 33143 (US)**

(74) Representative: **Eyles, Christopher Thomas et al**
**W.P. THOMPSON & CO. 50 Lincoln's Inn Fields**
**London WC2A 3PF (GB)**

(56) References cited:
**CHEMICAL ABSTRACTS, vol. 85, 1976, page 590, abstract no. 63304c, Columbus, Ohio, US; & JP - A - 76 36 467 (TANABE SEIYAKU CO., LTD.) 27-03-1976**
**CHEMICAL ABSTRACTS, vol. 69, 1968, page 3111, abstract no. 33385g, Columbus, Ohio, US; I.I. PELEVINA et al.: "Radiosensitizing action of halo-substituted thymidine analogs, & IZV.**
**AKAD. NAUK SSSR, SER. BIOL. 1968, (3), 345-52**
**J. Am. Chem. Soc. Cummunication to the Editor Vol. 81, Published 5 Aug. 1959 Berger Simple Synthesis of Pyrimidine-2-Deoxy Ribonucleosides pp- 4112-13**
**Chemotherapy of Cancer, Second Edition page 9, published 1981 (New York, N.Y. U.S.A.) Carter et al**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 160 079 B1

**Description**

The present invention pertains to combination radiotherapy of tumors and more specifically to pharmaceutical compositions for use in methods of treatment and therapy designed to sensitize tumors in animals, notably humans, and render them more sensitive to radiation, thus significantly reducing the amount of radiation required to kill neoplastic cells while at the same time making the radiation far more tissue specific to the tumor site.

More conventional radiation sensitizers are hypoxic cell sensitizers such as the antifilarial agent, misonidazole, which causes the complexities of neurotoxicity when it is utilized in humans. The 5-halo-generated pyrimidine analogs are very distinct agents from the hypoxic cell sensitizers, for they have a completely different mode of action. A review entitled "X-Ray Sensitization by Halopyrimidines" by Waclaw Szybalski appeared in Cancer Chemotherapy Reports, Part 1, Vol 58, No. 4, July/August 1974, at page 539 et seq. We have found that cultured mammalian cells, when exposed to bromo-2'-deoxyuridine (BrdU) or other halogenated analogs of thymidine incorporate the compound into DNA resulting in sensitization of these cells to X-irradiation. Rapid catabolism or degradation of BrdU has limited its clinical effectiveness for the sensitization of rapidly growing neoplasias.

We have determined that 5-chloro-2'-deoxycytidine (CldC), which is not readily degraded due to the 4-amino group that protects the compound from catabolism by nucleoside phosphorylases, is anabolized by a different set of enzymes than the corresponding dU analog. In addition CldC is less cytotoxic than CldU. An object of the present invention is to define a class of stable, selective cell sensitizers that are not easily catabolized against tumor, both rapidly and moderately growing and malignant, and that will allow the X-ray therapist to focus the X-ray beam (or other source of radiation) on the tumor tissue site using significantly less, say one-fourth, of the dose of radiation otherwise required to achieve the same extent of tumor kill without damage to the underlying tissue. Expressed in another way, the radiotherapist is able to more aggressively kill neoplastic tissue while causing no more damage to normal tissue using the procedures and materials of the present invention than with conventional modalities of irradiation.

Thus an object of the present invention is to provide therapeutic materials for treating skin lesions using, for instance, ultraviolet light, near visible light (313 nm), and for solid tumors: X- or gamma ray, beta, neutron and other radiation entities.

Another object of the invention is to provide materials which can sensitize possible sites of metastatic invasion to radiation, particularly X-ray.

Should the patient develop toxicity from the mildly aggressive therapy employed, particularly with the pretreatment with FdU and PALA either before or with the sensitizing composition, thymidine or deoxycytidine, two non-toxic metabolites, may be provided the patient at the conclusion of radiation therapy. These serve to mitigate the untoward effects, if any, of the drug therapy by antagonizing or reversing any toxic effects of the chemotherapeutic agents employed.

The pharmaceutical compositions of the present invention may be used primarily with X-ray or gamma (derived from cobalt, for example) radiation. We also consider them to be effective with the use of ultraviolet light, near visible light (313 nm) for skin lesions and for beta, neutron and other radiation entities. The molecular basis of sensitization has been clearly established for ultraviolet light (260 nm) and near visible light (313 nm) (both nonpenetrating) and for X- or gamma-radiation.

Summary of the Invention

In accordance with the present invention there is provided the use for the manufacture of a pharmaceutical composition for sensitizing neoplastic tissue to radiation of a compound of the formula:

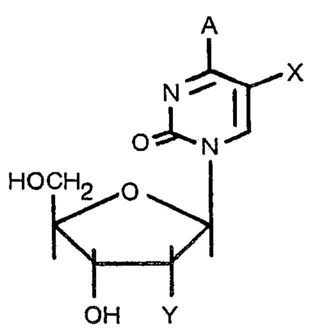

(I)

wherein A is —N $H_2$; X is chloro; and Y is hydrogen, chloro, bromo, iodo or fluoro.

The invention further provides products containing a compound of the formula (I) and a deamination inhibitor selected from tetrahydrouridine ($H_4U$) and/or 2'-deoxytetrahydrouridine ($dH_4U$), for simultaneous, separate or sequential use for sensitizing neoplastic tissue to radiation.

In a preferred aspect of the invention there are provided products containing

(1) at least one pretreatment agent; and

(2) (a) 5-chloro-2'-deoxycytidine or 5-chloro-2'-halo-2'-deoxycytidine wherein halo is fluoro, chloro, bromo, or iodo, in admixture with

(b) tetrahydrouridine and/or 2′-deoxytetrahydrouridine, for sequential use for sensitizing susceptible neoplastic tissue to radiation, said pretreatment agent (1) being selected from 5-fluoro-2′-deoxyuridine; 5-fluoro-2′-deoxycytidine plus tetrahydrouridine; N-(phosphonacetyl)-L-aspartate; and combinations thereof.

In a further preferred aspect of the invention there is provided a pharmaceutical composition for sensitizing neoplastic tissue to radiation comprising a radiation sensitizing effective amount of a deoxycytidine compound selected from 5-chloro-2′-deoxycytidine and 5-chloro-2′-halo-2′-deoxycytidine, wherein halo is fluoro, chloro, bromo or iodo, together with a deamination inhibiting effective amount of a deamination inhibitor selected from tetrahydrouridine ($H_4U$) and/or 2′-deoxytetrahydrouridine ($dH_4U$).

Patients having tumors requiring radiation therapy can be administered, preferably on a slow release basis, 5-chloro-2′-deoxycytidine and/or 5-chloro 2′-halo-2′-deoxycytidine, wherein halo is fluoro, chloro, bromo or iodo, preferably chloro. The deoxycytidine compound is preferably administered with a deamination inhibitor, preferably tetrahydrouridine ($H_4U$) and/or 2′-deoxytetrahydrouridine ($dH_4U$) for a period of time until amounts sufficient to sensitize the tumor tissue to radiation are present in the tumor tissue. Optimally, the patient is given a pretreatment regimen designed to lower the metabolities competing for CldC, or a special limited diet is used for the same purpose. Drug treatment is suspended and radiation therapy initiated at the dosage required to kill the exposed tumor tissue while avoiding or reducing significant damage to the underlying tissue. If toxicity is encountered in the pretreatment sensitization procedure, thymidine or deoxycytidine may be administered to the patient immediately following the radiation treatment to counteract toxicity without affecting selective tumor kill.

Pharmaceutical compositions providing the required amounts of 5-Cl-2′-halo-2′-dC as well as compositions providing the required amounts of 5-CldC and $H_4U$ or $dH_4U$ in pharmaceutically acceptable formulations are also described.

Rapid catabolism and generalized toxicity have limited the use of 5-halogenated analogs of deoxyuridine as tumor sensitizers. In one approach to this problem, 5-halogenated analogs of deoxycytidine (dC) or of 2′-halo 2′-deoxycytidine were utilized, which are not catabolized unless they are deaminated. To prevent deamination by cytidine deaminase (CD), which is extremely active in human serum, it is preferred, according to the invention, to administer tetrahydrouridine ($H_4U$), a potent inhibitor of this enzyme, either concurrently with, or at about the same time as, administration of the deoxycytidine compound.

Our previous enzyme kinetic studies with 5-bromo-2′-deoxycytidine (BrdC) and 5-iodo-2′-deoxycytidine (IdC) indicate that they would not be suitable, in this approach to circumvent catabolism, because they are poor substrates for deoxycytidine kinase. Unlike BrdC, chlorodeoxycytidine (CldC) does not require deamination at the nucleoside level for its anabolism because it possesses a reasonable Km value (56 M) with respect to mammalian deoxycytidine kinase compared to 400 M for BrdC and 2 M for dC. Studies with HEp-2 cells suggest that CldC (+$H_4U$) is metabolized as follows: ClDC $\xrightarrow{1}$ CldCMP $\xrightarrow{2}$CldUMP $\xrightarrow{3}$ CldUTP $\xrightarrow{4}$DNA (1=deoxycytidine kinase, 2=deoxycytidylate deaminase (dCMPD), 3-thymidylate kinase, (4-DNA polymerase). These four enzymes are elevated in many human tumors. For example, dCMPD activity in human malignant tumors is 20—80 fold higher than that of normal tissue.

In X-radiation studies with HE-2 cells, we have obtained 3.4—3.7-fold does increase effects. Cells were preincubated with inhibitors of *de novo* pyrimidine synthesis: N-(Phosphonacetyl)-L-aspartate (PALA) and 5-fluorodeoxyuridine (FdU) for 20 hours and 5 hours, respectively and then incubated in the presence of 0.1 or 0.2 mM CldC and $H_4U$ (100 M) for 64 hours. These conditions result in 40—50% substitution of CldU for thymidine in DNA. Viabilities of 10% ± 4 to 12% ± 5 were obtained for drug-treated unirradiated cells. Inhibitors of thymidylate synthetase that are more DNA and tumor selective than FdU are also within the ambit of this invention. CldC and its metabolites are not toxic unless deamination occurs. CldC should be converted preferentially to CldUMP in tumors possessing high levels of dCMPD and then be further anabolized to CldUTP, resulting not only in radiosensitization but also in selective tumor toxicity, presumably as a result of inhibition of ribonucleoside diphosphate reductase by CldUTP.

Addition of $dH_4U$, which results in inhibition of CD *and* dCMPD, has enabled us to study radiosensitization due to incorporation of CldC as such into DNA.

The preferred materials used to carry out the present invention, including abbreviations and structural formulae, are listed in Table I below. 5-chlorodeoxycytidine (CldC) was obtained from Calbiochem-Behring and has been described in the literature as an antiviral (antiherpetic); see Fox, Mekras, Bagwell and Greer et al, Capacity of Deoxycytidine to Selectively Antagonize Cytotoxicity of 5-Halogenated Analogs of Deoxycytidine Without Loss of Aniherpetic Activity, Antimicrob. Agents Chemother., Vol. 22, No. 3, p. 431—441 (Sept. 1982). Additionally DeClercq et al used CldC in cell culture studies (no indication is given for use in cancer therapy) the data indicating CldC was unremarkable in the system employed; see "Role of Deoxycytidine Kinase in the Inhibiting Activity of 5-substituted 2′-Deoxycytidines and Cytosine Arabinosides on Tumor Cell Growth", J. Balzarini, and DeClercq, Erik, Molecular Pharmacology, Vol. 23, p. 175—181 (1982).

The 5-chloro-2′-halo-2′-deoxycytidines may be prepared according to the procedure described by Codington, Doerr and Fox, Nucleosides, XVIII Synthesis of 2′-Fluorothymidine, 2′-Fluorodeoxyuridine, and other 2′ halogeno-2′-deoxy Nucleosides, J. Org. Chem., 29, 558 (1964).

Tetrahydrouridine ($H_4U$) was obtained as a gift from the Drug Development Branch of the National

Cancer Institute, Bethesda, Maryland, its synthesis is described by Hanze, Catalytic Reduction of Pyrimidine Nucleosides, J. Amer. Chem. Soc., *89* 6720—6725 (1967). 2′-deoxytetrahydrouridine ($dH_4U$) inhibits cytidine deaminase and when phosphorylated it also inhibits deoxycytidylate deaminase. The synthesis of $H_4U$ and $dH_4H$ are described in U.S. 4,017,606 (Hanze et al). To our knowledge $dH_4H$ has never been utilized in tumor therapy with an analog of deoxycytidine.

5-fluorodeoxyuridine (FdU) is a known antitumor agent available from Sigma Chemical Company, 5-fluorodeoxycytidine (FdC), which has a greater selectivity against tumors, may also be used. FdC, not previously been used for tumor radiosensitization, may be prepared according to Fox, J.J., Wempen, I., and Duschinsky, R., Nucleosides of 5-Fluorocytosine. Proc. of the 4th International Congress of Biochemistry *15* p. 6 (1958). For the procedure of the present invention it is co-administered with tetrahydrouridine.

N-(Phosphonacetyl)-L-aspartate (PALA) has been used alone and with 5-fluroruracil (5-FUra) as an antitumor agent but not to pretreat tumor cells in conjunction with radiation. PALA was obtained from the Drug Development Branch of the National Cancer Institute at Bethesda, Maryland.

The strategy of pretreatment is to inhibit the de novo pathway of pyrimidine biosynthesis.

The use of 5-trifluoromethyl-2′-deoxycytidine ($F_3$methyldC) together with tetrahydrouridine ($H_4U$) in the treatment of Herpes or Herpes-like viruses is described in U.S. 4,210,638 to Sheldon Greer.

TABLE I

| Name | Abbreviation | Structure |
|---|---|---|
| tetrahydrouridine | $H_4U$ | 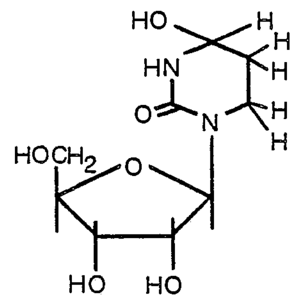 |
| 2′-deoxytetrahydrouridine | $dH_4U$ | 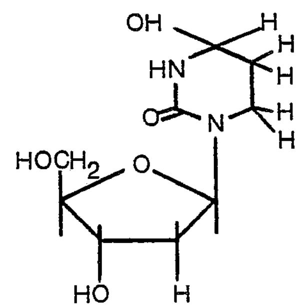 |
| 5-chloro-2′-deoxycytidine | 5-CldC | 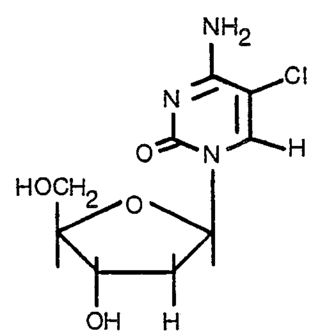 |
| 5-fluoro-2′-deoxyuridine | FdU | 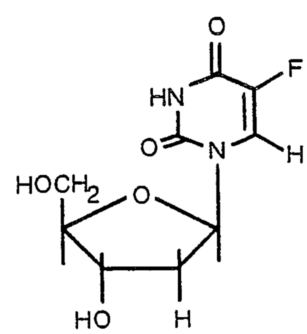 |
| 5-fluoro-2′-deoxycytidine | FdC | |

TABLE I

| Name | Abbreviation | Structure |
| --- | --- | --- |
| N-(Phosphonacetyl)-L-aspartate | PALA 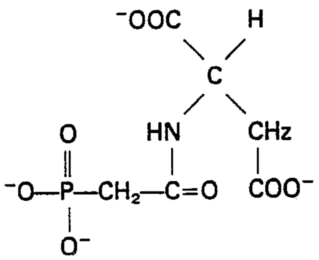 | 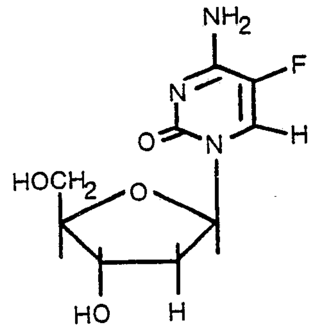 |

The abbreviations used include: CD, cytidine-deoxycytidine deaminase; CHO, Chinese hamster ovary cells; CldC, 5-chloro-2'deoxycytidine; CldCMP, 5-chloro-2'-deoxycytidine-5'-monophosphate; CldUMP, 5-chloro-2' deoxyuridine-5'-monophosphate; dC, deoxycytidine; dCK, deoxycytidine kinase; dCMPD, deoxycytidylate deaminase; $dH_4U$, 2'deoxytetrahydrouridine; dT, thymidine; dU, 2'-deoxyuridine; dUMP, 2'-deoxyuridine-5'monophosphate; FdC, 5-fluoro-2'-deoxycytidine; FdU, 5-fluoro-2'deoxyuridine; FdUMP, 5-fluoro-2'-deoxyuridine-5'-monophosphate; $F_3$methyldC, 5-trifluoromethyl-2'-deoxycytidine; FUra, 5-fluorouracil, HEp-2, human epidermoid laryngeal carcinoma cells, $H_4U$, tetrahydrouridine; TK, thymidine kinase; TS, thymidylate synthetase; TTP, thymidine-5'-triphosphate.

While not wishing to be bound by any theory we offer the following as a further explanation of the possible mode of action of CldC as a radiosensitizing agent when coadministered with $H_4U$ as well as with $dH_4U$. With a low concentration of tetrahydrouridine to protect the nucleoside analogs from systematic catabolism, one may envision that BrdC and IdC as well as CldC will act as selective radiosensitizers against tumors with high levels of cytidine deaminase. At higher concentrations of $H_4U$, CldC should be converted preferentially at the tumor site to CldUMP in human tumors possessing high levels of deoxycytidine kinase and dCMP deaminase. When anabolized to CldUTP not only will there be selective tumor toxicity because of inhibition of ribonucleoside reductase by CldUTP, but in addition the incorporation of CldU into DNA will lead to tumor radiosensitization. Selectivity will result not only because of accelerated DNA synthesis, but because of elevation of key enzymes in the tumor that are critical for this strategy; in addition, there will be the customary selectivity that is associated with utilizing a focussed beam of irradiation overlying the tumor. This approach is amenable to rescue with deoxycytidine and thymidine immediately after irradiation. A typical irradiation experiment in the mouse would involve an i.p. injection of CldC + $H_4U$ every 8 to 10 hours in a 30 to 36 period, for example. The goal is to obtain substantial incorporation of CldC into both strands of DNA. The use of $dH_4U$ may result in the incorporation of CldC as such into the DNA of cells. However, in this case there may be less selectivity against the tumor for we are probably only exploiting the elevation of dC kinase that occurs in tumors.

EXAMPLES OF THE INVENTION

To determine whether 5-chlorodeoxycytidine (CldC) and tetrahydrouridine ($H_4U$) has potential as a radiosensitizing combination, we tested these agents in HEp-2 cells. These cells were chosen as our model system since they possess elevated levels of activity of deoxycytidine kinase (dCK), cytidine deaminase (CD) and deoxycytidylate deaminase (dCMPD); the enzymatic profile necessary for metabolic conversion of CldC to an established radiosensitizer, CldUTP. TMP kinase and DNA polymerase are also elevated in tumors; these elevations should assure further preferential incorporation of CldU into tumor DNA.

Many of the experiments described below were done utilizing only one dose of radiation (usually 500 or 600 rads). Although one cannot accurately calculate dose-increase effects based on such limited data, we have nonetheless pursued our studies in this manner in order to test many different combinations of metabolites and antimetabolites in the same experiment. Naturally, we are missing important features seen only at low doses. However, this approach fulfilled the need to search and find optimal regimes. We have found that sensitizing effects are more pronounced at doses of 500 or 600 rads. Preliminary experiments that demonstrate some of the sensitization effects that have been obtained are described below.

CldC (and $H_4U$), our storage and DNA- and target-directed form of CldU, coadministered with methotrexate gave an enhancement ratio of 2.0-fold. This is illustrated in Figure 1. This effect was obtained with a viability of 66%.

In the next series of experiments, cells were pretreated prior to CldC + $H_4U$ administration with the inhibitor of *de novo* pyrimidine biosynthesis, N-(Phosphonacetyl)-L-aspartate (PALA). PALA is a potent inhibitor of aspartate transcarbamylase and causes a depletion of intracellular pyrimidine pools. *Liang et al* found that PALA and fluorouracil (FUra) were synergistic when PALA was administered prior to FUra. These investigators speculated that the reason for this synergistic interaction was due to marked decreases in dUMP pools in cells preincubated with PALA. Decreased dUMP pools should help potentiate FdUMP inhibition of thymidylate synthetase leading to decreased levels of TTP; which would then result in less competition for the incorporation of CldU into DNA and greater activity of dCMPD. This is the rationale for the use of the combination of PALA and FdU in radiation experiments. Furthermore, PALA, by reducing intracellular pyrimidine biosynthesis may lower competing substrates for the activation of CldC to CldUTP.

In recent experiments PALA has been administered 12—20 hours prior to FdU pretreatment, which is for 6 hours. *Evans et al* have shown that FdUMP pools persist after FdU or FUra administration. Coexposure of HEp-2 cells to CldC + $H_4U$ and FdU for 48 hours in comparison to pretreatment with FdU for 6 hours does not lead to a greater enhancement of radiosensitization by CldC + $H_4U$ but only results in greater cytotoxicity. Following the pretreatment schedule described above, we have been able to lower the concentration of CldC (from 0.6 mM to 0.2 mM) and achieve better sensitization to X-ray. These results are illustrated in Figure 2. CldC at 0.2 mM gives a dose increase effect of 3.6 with an associated 12.4% ± 5.1% (± S.E.) viability, whereas CldC at a concentration of 0.6 mM results in a 3.0-fold dose increase.

In the experiment summarized in Figure 3 we attempted to lower the concentration of CldC further, but lost sensitization at a concentration of 0.05 mM. In an effort to minimize the number of manipulations performed, cells were exposed to PALA and FdU for 21 hours as a single pretreatment, but this resulted in a significant loss in viability (viability equals 1.6%) with no gain in radiosensitization (a 1.9 dose-increase). A 3.8-fold dose increase effect was achieved with conditions similar to those of the previous experiment with 9.8% ± 4.0% (± S.E.) viability.

The rationale for utilizing PALA and FdU pretreatment is to achieve greater radiosensitization with CldC + $H_4U$; however our approach is strengthened by the fact that PALA and fluorinated pyrimidines are agents which are effective in combination chemotherapy. The conversion of CldCMP to CldUMP at the tumor site because of elevated levels of dCMP deaminase, is an example of tumor-directed toxicity as is the case with FdC. The target enzyme in CldC therapy is presumably nucleoside diphosphate reductase, which is likely inhibited by CldUTP. FdC pretreatment rather than FdU is currently believed to achieve a greater measure of tumor- and DNA-directed toxicity with no loss of radiosensitization with CldC, $H_4U$ and PALA in animal systems.

The experiment summarized in Figure 4 illustrates our second approach with CldC; that is, to examine the radiation effects resulting from the incorporation of CldC *as such* (without prior deamination to CldU) in DNA. This may be accomplished by $dH_4U$, which as $dH_4UMP$ inhibits both deoxycytidine and deoxycytidylate deaminases. If both sites of deamination are blocked, the only anabolic route for CldCMP will be to become phosphorylated further to CldCTP. In this initial experiment a 1.8-fold dose-increase effect was obtained with CldC and $dH_4U$.

In subsequent experiments we sought to lower competing dCTP pools by utilizing 3-deazauridine. Deazauridine is a potent inhibitor of CTP synthetase and did not appear to enhance sensitization in this experiment. In the bar graph depicted in Figure 5 a 2.0-fold dose-increase effect was obtained by CldC, $dH_4U$ and deazauridine with 21% viability.

It should be noted that the most striking effects we have obtained have been with the use of CldC and $H_4U$ rather than with CldC + $dH_4U$. That is, the combination of CldC + $H_4U$ has resulted in a 3.4 to 3.8 dose enhancement effect with appropriate pretreatment (PALA and an F-pyrimidine analog).

Figure 6 summarizes an experiment in which a 3.4 dose enhancement effect is displayed when FdC + $H_4U$ replaces FdU in the pretreatment procedure prior to the addition of CldC and $H_4U$. 5-fluorodeoxy-cytidine + tetrahydrouridine should result in tumor directed toxicity; that is, it should be more tumor specific than FdU. Thus FdC + $H_4U$ may be used to obtain greater efficacy without loss of radiosensitization.

Figure 7 summarizes an experiment in which we have demonstrated that lowering *both* PALA and CldC concentrations results in significant loss of radiosensitization without a substantial decrease in toxicity. FdU and FdC + $H_4U$ pretreatment are essentially equivalent in terms of effective CldC radiosensitization, with FdC + $H_4U$ displaying less toxicity, which is desirable. A maximum 3.4 to 3.6 dose enhancement effect was displayed in this experiment.

BrdU has been shown under the most optimal conditions in cell culture to display a 3.5 to 4.0 dose-increase effect with X-ray and with ultraviolet light. We have now achieved comparable results with a combination of agents that will lead to the circumvention of catabolism and to tumor selectivity — two features not readily achieved with BrdU. Most importantly, with this method one may irradiate a tumor at 1/4 the dose to prevent damage to underlying tissue or to more aggressively irradiate a tumor without an increase in damage to normal tissue.

*Pharmaceutical Presentation* — pharmaceutical compositions comprising, as the active ingredient(s), radiation-sensitizing effective amounts of 5-CldC and $H_4U$ and/or $dH_4U$ together with a pharmaceutically acceptable carrier or diluent, for intraperitoneal administration for animal studies, intraveneous, subcutaneous, intramuscular, oral or topical administration are included in the present invention. While the

components of the composition may be administered separately, it is preferred to coadminister them as a mixture. The concentration of each of the active ingredients may vary from about 0.01 to about 25% by weight depending on the route of administration, the frequency of administration, the severity of the condition, the age, weight and general physical condition of the patient being treated as well as the size and location of the tumor to be irradiated. Alternatively a more concentrated solution will be used, e.g. 75 g/100 ml, or a slow i.v. infusion of a 0.1 to 25% (or higher) concentration will be used. When the composition is in the form suitable for topical administration, for example a cream, the concentration of the total of 5-CldC and $H_4U$ or $dH_4U$ will generally vary from about 5 to 50 wt.%, preferably about 5 to 20 wt.%, more preferably from about 5 to 10 wt.%. When the composition is in the form suitable for intraperitoneal administration for animal studies, for example, an aqueous solution of CldC and $H_4U$ or $dH_4U$ the concentration will generally vary from about 0.5 to 5% w/v, more usually from 1% w/v. For oral administration, the concentration will generally be from 0.05 to 10 wt.%, preferably about 0.5 to 5 wt.%, and more preferably about 1 to 2 wt.%.

When used for intravenous injection, the concentration of the active components will vary from about 0.05 to about 5% w/v, preferably about 0.1 to about 0.5% w/v. For intramuscular injection, the same concentration as described above for the intraperitoneal mode of administration will be utilized.

Other methods of administration may also be used. Suppositories may be used for sustained release purposes. Slow-release surgical implants are also envisaged.

The pharmaceutically acceptable carriers or diluents employed in the compositions of the present invention may be any compatible non-toxic material suited for mixing with the active compounds. When the composition is in a form suitable for parenteral use, for example intramuscularly or intravenously, the carrier which preferably is an aqueous vehicle, may also contain other conventional additives, such as a suspending agent for example methyl cellulose or polyvinylpyrrolidone (PVP), and a conventional surfactant. For oral administration, the compositions can be formulated as aqueous solutions, suspensions, capsules or tablets, suitably containing appropriate carriers or diluents, for example lactose, starch and/or magnesium stearate for flavoring agents, syrups, sweeteners or coloring materials as customarily used in such preparations.

A preferred pharmaceutical composition provides the patient with a total i.v. dosage of from 3 to 5 ml (cc) per dosage calculated on 70 kg body weight of the patient.

*Clinical Protocol*: The patient will be given drugs i.v. or i.m. or in an oral or suppository form or in a slow release form. A slow release administration of CldC and tetrahydrouridine may be particularly advantageous. Different routes may be used for individual drugs in one treatment protocol.

PALA in 10 ml ampules containing PALA disodium (1.0 gram) with Edetate disodium (1 mg) and NaOH to adjust pH to 6.5 to 7.5 will be given to a cancer patient with a solid tumor at a range 2 mg to 300 mg/kg per dose preferably 5 to 20 mg/kg per dose and more likely 10 mg/kg per dose. Twelve to thirty-six hours later preferably 18 to 26 and most likely 24 hours later, FdU at a concentration of 10—75 mg/kg per dose, preferably 25 to 60 more likely 50 mg/kg would be administered. Alternatively FdC at similar concentration range as FdU will be administered but in this case it will be coadministered with tetrahydrouridine at a concentration range from 10 to 200 mg/kg preferably 15 to 100 and more likely 25 mg/kg. The ratio of $H_4U$ to FdC will range from 4:1 to 0.2:1 preferably 1.5:1 to 0.75:1 more likely 1:1.

Three to 12 hours later preferably 4 to 8 hours, more likely 6 hours later the series of administration of 5-CldC and $H_4U$ will begin.

The dose of 5-CldC will range from 200 to 2500 mg/kg per dose, preferably 500 to 2000 mg/kg, more likely 1500 mg/kg.

The dose of $H_4U$ will be the same as that given with FdC described above. The ratios are, however, different; namely, the ratio of $H_4U$ to CldC will range from 1:30 to 1:5, more usually 1:30 to 1:10 and more likely 1:15.

This will be repeated at 6 to 18 hr intervals more usually 8 to 12 more likely 10 hr intervals. The period of repeated CldC + $H_4U$ administration will be 20 to 60 hrs, more usually 30 to 50 hrs more likely 34 to 48 hrs. Usually CldC + $H_4U$ will be administered in 3 to 4 doses, 8 to 12 hrs apart.

After the last dose of CldC + $H_4U$ an interval of 4 to 8 hrs will ensue prior to irradiation. This period will more preferably be 6 to 14 hrs and more likely 8 to 10 hrs.

The interval between PALA/FdU or FdC antitumor therapy and CldC/$H_4U$ sensitizer therapy, as well as the frequency of administration is determined by the skilled clinician drawing upon previous experiences and observations using this regimen and therapy. The interval between drug therapy and radiation treatment may be varied as well.

The radiation dose, X-ray or gamma-ray, for example, will be either the same or 1/4 to 3/4 the dose given to patients not receiving the pretreatment sensitization schedule. This will result in either (a) more aggressive tumor kill without increased damage to underlying tissue when the 4/4 dose is used, or (b) equal tumor kill as that achieved in patients given no treatment but with much less damage to underlying tissues when the 1/4 to 3/4 doses are used.

If toxicity is encountered due to the drug treatment schedule then thymidine at a dose of 50 to 750 mg/kg, more likely 100 to 500 mg/kg, preferably 200 mg/kg will be given immediately after the radiation treatment. This will be repeated 2 to 3 times at 8 to 12 hr intervals. This treatment is designed to counteract toxicity without adversely affecting selective tumor kill. Deoxycytidine at a concentration range similar to

thymidine can be given with or instead of thymidine. When deoxycytidine is administered it will be given at a ratio of tetrahydrouridine to deoxycytidine of 1:.05 to 1:5.*

This course of treatment can be repeated one week to two weeks later and repeated again until the patient receives a total dose of 3000 to 7000 rads. In this strategy, the patient may need less total irradiation to achieve effective tumor kill. This is one advantage of the strategy. The dose of radiation which, in the past provided only partial remission, may result in long term cures. That is the primary advantage of this approach.

The dosages and ranges are summarized in the following table:

TABLE II
DOSAGE**

| Agent | | General | Preferred | Most Preferred |
|---|---|---|---|---|
| pretreating | PALA | 2—300 | 5—20 | 10 |
| | FdU or FdC | 10—75 | 25—60 | 50 |
| | + $H_4U$*** | 10—200 | 15—100 | 25 |
| Sensitizing | CldC | 200—2500 | 500—2000 | 1500 |
| | $H_4U$ | 10—200 | 15—100 | 25 |
| | $dH_4U$ | 10—200 | 25—125 | 50 |

* Deoxycytidine with $H_4U$ or $dH_4U$ also be given 2—6 hrs after each CldC treatment prior to irradiation.
** expressed in mg/kg body weight/dose
*** when FdC is utilized

The above dosages and ranges apply with respect to the 5-chloro-2′-halo-2′-deoxycytidine compounds.

*Toxicity Studies*: Our analysis of the potential of toxicity using the method herein disclosed indicates that, at most, a 5% weight loss was achieved with no deaths due to toxicity. This is viewed as a trivial and most tolerable weight loss considering the normal aggressive results of the administration of antitumor agents in radiation therapy.

The protocol employed was as follows: Three animals were injected i.p. with PALA at a dose of 200 mg/kg. This was followed 24 hrs later with an i.p. injection of 5-fluorodeoxyuridine (FdU) at a dose of 50 mg/kg. Four hrs later they were given an i.p. injection of CldC (500 mg/kg) coadministered with 100 mg/kg tetrahydrouridine. The administration of CldC + $H_4U$ at the above indicated concentrations was repeated two more times at ten hour intervals. Only 4% weight loss occurred. No deaths occurred with this protocol.

This protocol was modified using 700 and 1400 mg of CldC/kg and a FdU concentration of 60 mg/kg and extensive incorporation of 5-chlorodeoxyuridine into DNA of tumor tissue was observed.

The procedures of our invention go beyond taking advantage of the rapid growth of tumors. It exploits important quantitative differences in the levels of enzymes between neoplastic and normal tissue. CldUTP, a metabolite product of CldC when administered in the presence of $H_4U$ is preferentially formed in tumor tissue to result in tumor directed toxicity and radiosensitization. Because the mode of radiosensitization of pyrimidine analogs differs from that of hyperthermy and hypoxic cell sensitizers, our procedure and strategy may be used with those modalities in radiation therapy.

**Claims**

1. The use for the manufacture of a pharmaceutical composition for sensitizing neoplastic tissue to radiation of a compound of the formula:

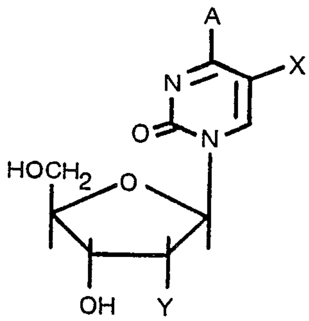

(I)

wherein A is —$NH_2$; X is chloro; and Y is hydrogen, chloro, bromo, iodo or fluoro.

2. Products containing a compound of the formula (I) set out in claim 1 and a deamination inhibitor selected from tetrahydrouridine ($H_4U$) and/or 2'-deoxytetrahydrouridine ($dH_4U$), for simultaneous, separate or sequential use for sensitizing neoplastic tissue to radiation.

3. Products according to claim 2, in which the ratio of the $H_4U$ and/or $dH_4U$ to the deoxycytidine compound is in the range of about 1:30 to 1:5.

4. Products containing a pretreatment agent and a compound of the formula (I) set out in claim 1 for sequential use for sensitizing neoplastic tissue to radiation, said pretreatment agent being selected from 5-fluoro-2'-deoxyuridine; 5-fluoro-2'-deoxycytidine plus tetrahydrouridine; N-(phosphonacetyl)-L-aspartate; and combinations thereof.

5. Products containing

(1) at least one pretreatment agent; and

(2) (a) 5-chloro-2'-deoxycytidine or 5-chloro-2'-halo-2'-deoxycytidine wherein halo is fluoro, chloro, bromo, or iodo, in admixture with

(b) tetrahydrouridine and/or 2'-deoxytetrahydrouridine, for sequential use for sensitizing susceptible neoplastic tissue to radiation, said pretreatment agent (1) being selected from 5-fluoro-2'-deoxyuridine; 5-fluoro-2'-deoxycytidine plus tetrahydrouridine; N-(phosphonacetyl)-L-aspartate; and combinations thereof.

6. Products according to claim 5, in which the ratio of (b) to (a) is in the range of about 1:30 to 1:5.

7. Products according to claim 5 or claim 6, in which (a) is 5-chloro-2'-chloro-2'-deoxycytidine.

8. Products according to claim 7, further including deoxycytidine with or without a deamination inhibitor and/or thymidine for administration after irradiation to rescue normal cells, said deamination inhibitor being selected from tetrahydrouridine ($H_4U$) and deoxytetrahydrouride ($dH_4U$).

9. A pharmaceutical composition for sensitizing neoplastic tissue to radiation comprising a radiation sensitizing effective amount of a deoxycytidine compound selected from 5-chloro-2'-deoxycytidine and 5-chloro-2'-halo-2'-deoxycytidine, wherein halo is fluoro, chloro, bromo or iodo, together with a deamination inhibiting effective amount of a deamination inhibitor selected from tetrahydrouridine ($H_4U$) and/or 2'-deoxytetrahydrouridine ($dH_4U$).

10. A composition according to claim 9, in which the deoxycytidine compound is 5-chloro-2'-chloro-2'-deoxycytidine.

11. A composition according to claim 10, in which the weight ratio of tetrahydrouridine and 2'-deoxytetrahydrouridine to the deoxycytidine compound is from about 1:30 to 1:5.

**Patentansprüche**

1. Verwendung einer Verbindung der Formel:

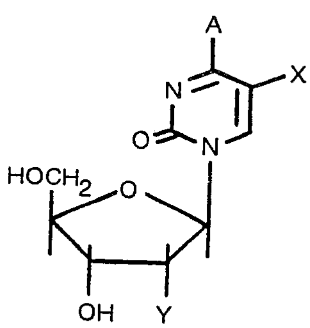

(I)

zur Herstellung einer pharmazeutischen Zusammensetzung zur Sensibilisierung von neoplastischem Gewebe gegenüber Strahlung, worin A —$NH_2$ darstellt, X Chlor und Y Wasserstoff, Chlor, Brom, Jod oder Fluor ist.

2. Produkte, die eine Verbindung der Formel (I) nach Anspruch 1 und einen Desaminierungsinhibitor enthalten, der aus Tetrahydrouridin ($H_4U$) und/oder 2′-Deoxytetrahydrouridin ($dH_4U$) ausgewählt ist, zur gleichzeitigen, separaten oder nachfolgenden Verwendung zur Sensibilisierung von neoplastischem Gewebe gegenüber Strahlung.

3. Produkte nach Anspruch 2, worin das Verhältnis von $H_4U$ und/oder $dH_4U$ zur Deoxyzytidinverbindung im Bereich von etwa 1:30 bis 1:5 liegt.

4. Produkte, die ein Vorbehandlungsmittel und eine Verbindung der Formel (I) nach Anspruch 1 enthalten, zur nachfolgenden Verwendung zur Sensibilisierung von neoplastischem Gewebe gegenüber Strahlung, wobei das Vorbehandlungsmittel aus 5-Fluor-2′-deoxyuridin, 5-Fluor-2′-deoxyzytidin plus Tetrahydrouridin, N-(Phosphonacetyl)-L-Aspartat und Kombinationen davon ausgewählt ist.

5. Produkte, die

(1) zumindest ein Vorbehandlungsmittel und

(2) (a) 5-Chlor-2′-deoxyzytidin oder 5-Chlor-2′-halogen-2′-deoxyzytidin, worin Halogen Fluor, Chlor, Brom oder Jod darstellt, in Vermischung mit

(b) Tetrahydrouridin und/oder 2′-Deoxytetrahydrouridin enthalten, zur nachfolgenden Verwendung zur Sensibilisierung von empfindlichem neoplastischem Gewebe gegenüber Strahlung, wobei das Vorbehandlungsmittel (1) aus 5-Fluor-2′-deoxyuridin, 5-Fluor-2′-deoxyzytidin plus Tetrahydrouridin, N-(Phosphonacetyl)-L-aspartat und Kombinationen davon ausgewählt ist.

6. Produkte nach Anspruch 5, worin das Verhältnis von (b) zu (a) im Bereich von etwa 1:30 bis 1:5 liegt.

7. Produkte nach Anspruch 5 oder 6, worin (a) 5-Chlor-2′-chlor-2′-deoxyzytidin ist.

8. Produkte nach Anspruch 7, die weiterhin Deoxyzytidin mit oder ohne Desaminierunginhibitor und/oder Thymidin enthalten, zur Verabreichung nach der Bestrahlung, um normale Zellen zu retten, wobei der Desaminierungsinhibitor aus Tetrahydrouridin ($H_4U$) und Deoxytetrahydrourid ($dH_4U$) ausgewählt ist.

9. Pharmazeutische Zusammensetzung zur Sensibilisierung von neoplastischem Gewebe gegenüber Strahlung, die eine für die Strahlungssensibilisierung wirksame Menge einer Deoxyzytidinverbindung, die aus 5-Chlor-2′-deoxyzytidin und 5-Chlor-2′-halogen-2′-deoxyzytidin ausgewählt ist, worin Halogen Fluor, Chlor, Brom oder Jod darstellt, zusammen mit einer zur Hemmung der Desaminierung wirksamen Menge eines Desaminierungsinhibitors umfaßt, der aus Tetrahydrouridin ($H_4U$) und/oder 2′-Deoxytetrahydrouridin ($dH_4U$) ausgewählt ist.

10. Zusammensetzung nach Anspruch 9, worin die Deoxyzytidinverbindung 5-Chlor-2′-chlor-2′-deoxyzytidin ist.

11. Zusammensetzung nach Anspruch 10, worin das Gewichtsverhältnis von Tetrahydrouridin und 2′-Deoxytetrahydrouridin zur Deoxyzytidinverbindung von etwa 1:30 bis 1:5 beträgt.

**Revendications**

1. Emploi pour la préparation d'une composition pharmaceutique, pour la sensibilisation aux radiations de tissus néoplasiques, d'un composé de formule:

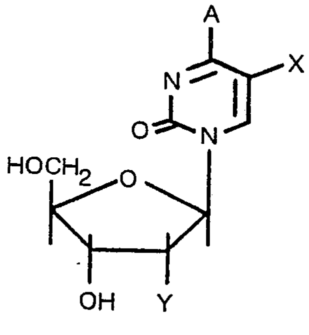

(I)

dans laquelle A est —$NH_2$; X est le chlore, et Y est l'hydrogène, le chlore, le brome, l'iode ou le fluor.

2. Produits contenant un composé de formule (I) mentionné dans la revendication 1 et un inhibiteur de désamination constitué par la tétrahydrouridine ($H_4U$) et/ou la 2′-désoxytétrahydrouridine ($dH_4U$) pour emploi simultané, séparé ou séquentiel afin de sensibiliser aux radiations des tissus néoplasiques.

3. Produits selon la revendication 2, dans lesquels le rapport de la $H_4U$ et/ou de la $dH_4U$ au composé de désoxycytidine est dans la gamme de 1:30 à 1:5 environ.

4. Produits contenant un agent de prétraitement et un composé de formule (I) mentionné dans la revendication 1 pour emploi séquentiel afin de sensibiliser aux radiations des tissus néoplasiques, cet agent de prétraitement étant choisi entre la 5-fluoro-2′-désoxyuridine, la 5-fluoro-2′-désoxycytidine plus la tétrahydrouridine, le N(phosphonacétyl)-L-aspartate, et des combinaisons de ces corps.

5. Produits contenant:

(1) au moins un agent de prétraitement, et

(2) (a) de la 5-chloro-2'-désoxycytidine ou de la 5-chloro-2'halo-2'-déoxycytidine dans laquelle l'halogène est le fluor, le chlore, le brome ou l'iode, en mélange avec

(b) de la tétrahydrouridine et/ou de la 2'-désoxytétrahydrouridine, pour emploi séquentiel afin de sensibiliser aux radiations des tissus néoplasiques sensibles, cet agent de prétraitement étant choisi entre la 5-fluoro-2'-désoxyuridine, la 5-fluoro-2'-désoxycytidine plus de la tétrahydrouridine, le N-(phosphonacétyl)-L-aspartate et des mélanges de ces corps.

6. Produits selon la revendication 5, dans lesquels le rapport de (b) à (a) est dans la gamme de 1:30 à 1:5 environ.

7. Produits selon la revendication 5 ou la revendication 6, dans lesquels (a) est la 5-chloro-2'-chloro-2'-désoxycytidine.

8. Produits selon la revendication 7, comprenant en plus de la désoxycytidine avec ou sans un inhibiteur de désamination et/ou de la thymidine pour administration après irradiation afin de secourir les cellules normales, cet inhibiteur de désamination étant choisi entre la tétrahydrouridine ($H_4U$) et la désoxytétrahydrouridine ($dH_4U$).

9. Composition pharmaceutique pour sensibiliser aux radiations des tissus néoplasiques comprenant une quantité efficace de sensibilisation aux radiations d'un composé de désoxycytidine choisi entre la 5-chloro-2'-désoxycytidine et la 5-chloro-2'-halo-2'-désoxycytidine, dans laquelle l'halogène est le fluor, le chlore, le brome ou l'iode, ensemble avec une quantité efficace pour inhiber la désamination d'un inhibiteur de désamination choisi entre la tétrahydrouridine ($H_4U$) et/ou la 2'-désoxytétrahydrouridine ($dH_4U$).

10. Composition selon la revendication 9, dans laquelle le composé de désoxycytidine est la 5-chloro-2'-chloro-2'-désoxycytidine.

11. Composition selon la revendication 10, dans laquelle le rapport en poids de la tétrahydrouridine et de la 2'-désoxytétrahydrouridine au composé de désoxycytidine est de 1:30 à 1:5 environ.

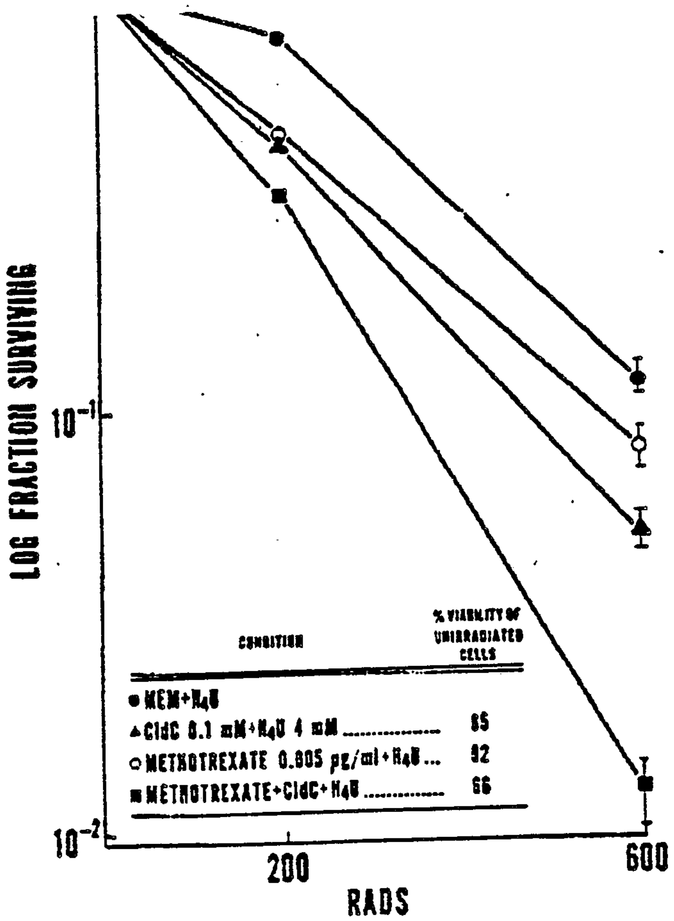
LOG FRACTION SURVIVING
$10^{-1}$
$10^{-2}$
200
600
RADS
CONDITION
% VIABILITY OF UNIRRADIATED CELLS
MEM+H4U
CldC 0.1 mM+H4U 4 mM 85
METHOTREXATE 0.005 µg/ml+H4U 92
METHOTREXATE+CldC+H4U 66

FIG. 1

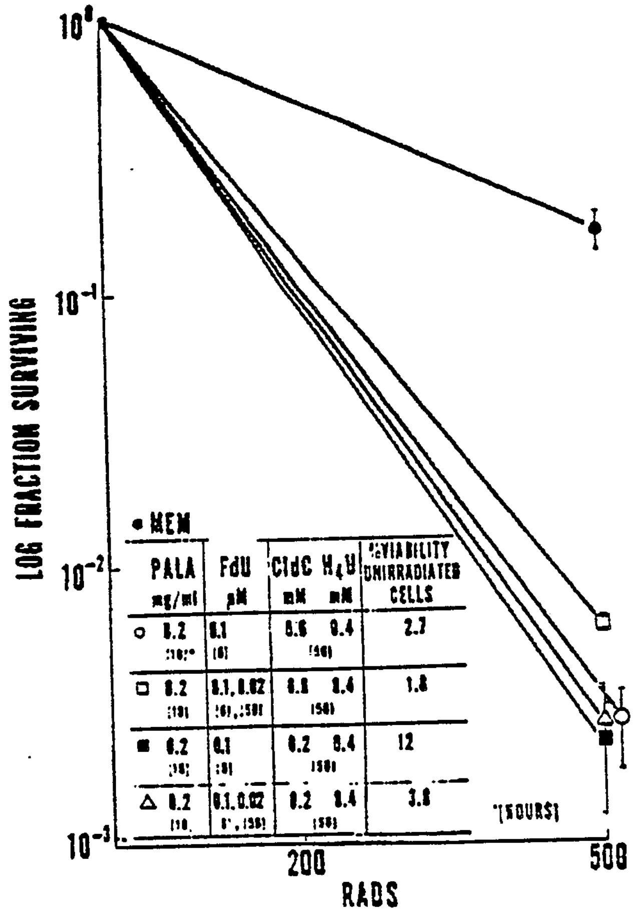
LOG FRACTION SURVIVING
$10^{0}$
$10^{-1}$
$10^{-2}$
$10^{-3}$
200
500
RADS
MEM
PALA mg/ml
FdU µM
CldC mM
H4U mM
% VIABILITY UNIRRADIATED CELLS
*(HOURS)

FIG. 2

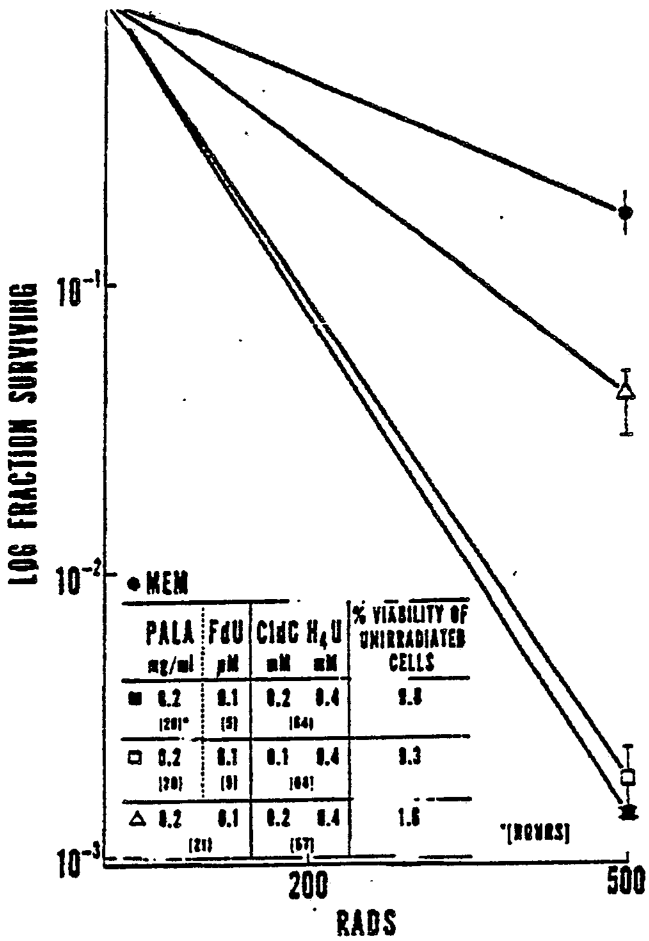
LOG FRACTION SURVIVING
$10^{-1}$
$10^{-2}$
$10^{-3}$
200
500
RADS
● MEM
PALA mg/ml
FdU µM
CldC mM
$H_4U$ mM
% VIABILITY OF UNIRRADIATED CELLS
*(HOURS)

FIG. 3

FIG. 4

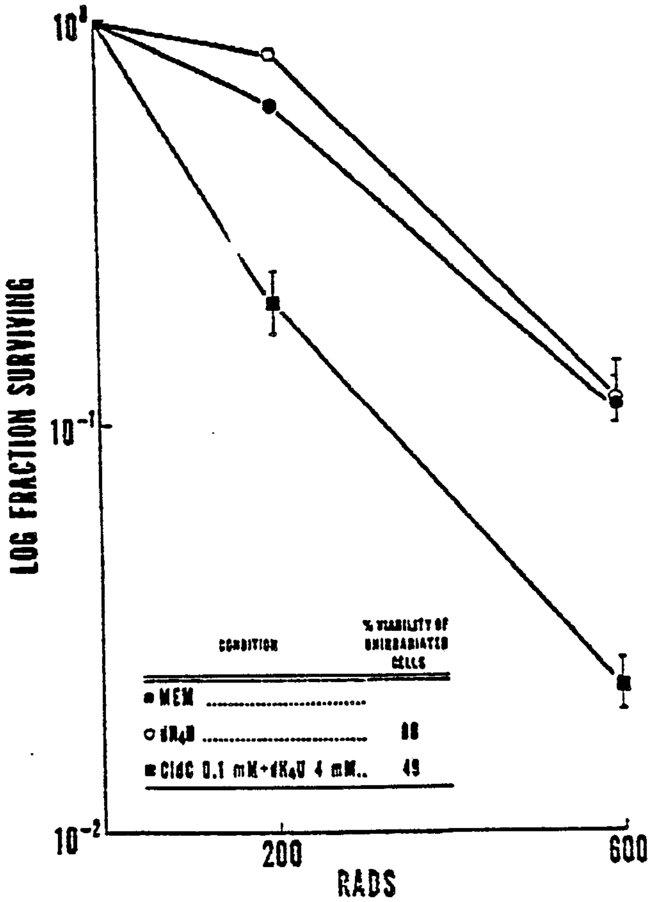
LOG FRACTION SURVIVING
$10^{0}$
$10^{-1}$
$10^{-2}$
200
600
RADS
CONDITION
% VIABILITY OF UNIRRADIATED CELLS
● MEM
■ CldC 0.1 mM + $dH_4U$ 4 mM.. 49

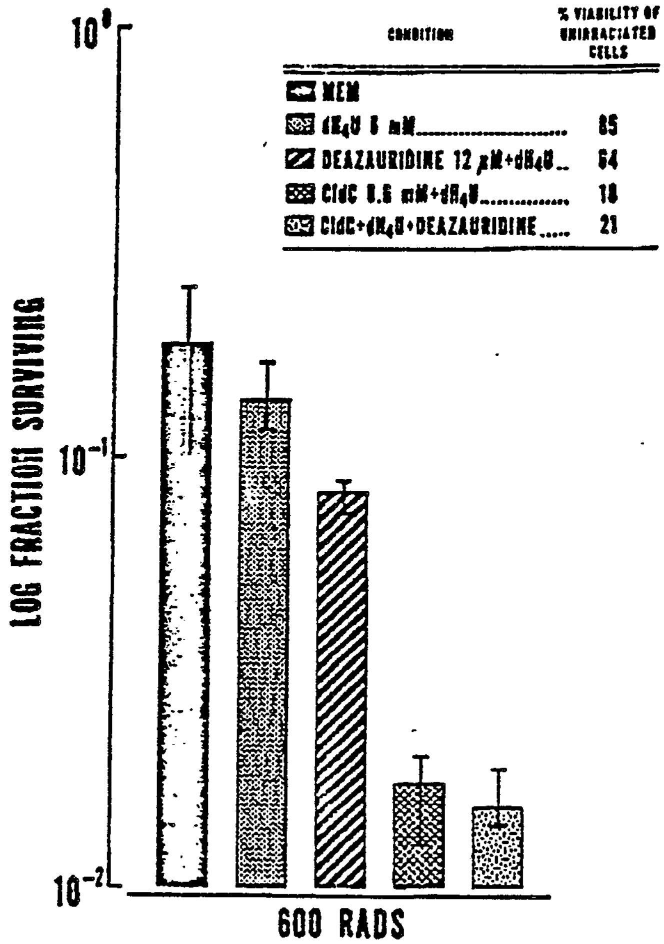
CONDITION
% VIABILITY OF UNIRRADIATED CELLS
MEM
dH4U 5 mM 85
DEAZAURIDINE 12 μM+dH4U 64
CldC 0.5 mM+dH4U 18
CldC+dH4U+DEAZAURIDINE 21
LOG FRACTION SURVIVING
10^0
10^-1
10^-2
600 RADS

FIG. 5

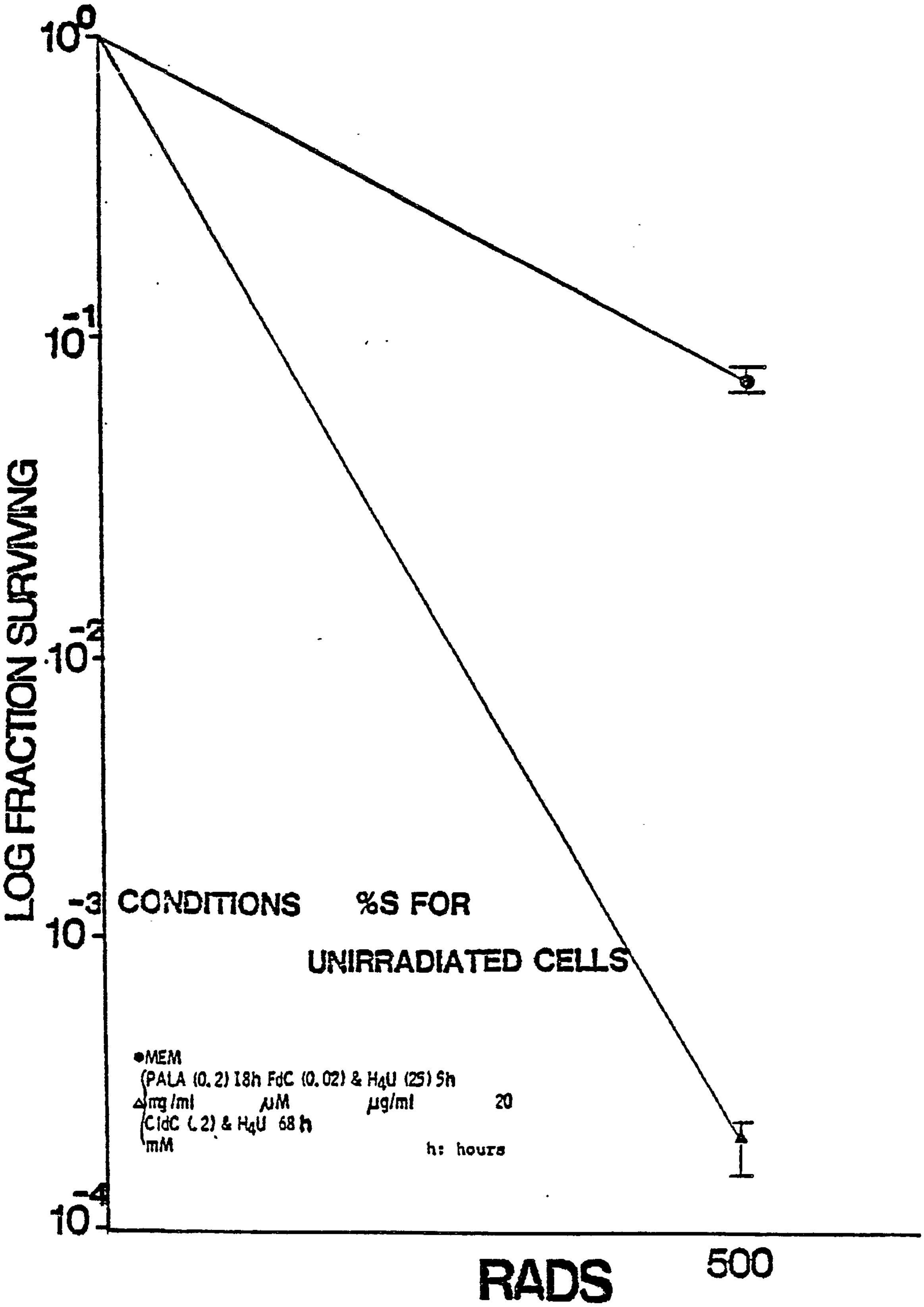
10^0
10^-1
10^-2
10^-3
10^-4
LOG FRACTION SURVIVING
CONDITIONS %S FOR
UNIRRADIATED CELLS
●MEM
PALA (0.2) 18h FdC (0.02) & H4U (25) 5h
△ mg/ml μM μg/ml 20
CldC (.2) & H4U 68 h
mM
h: hours
RADS
500

FIG. 6

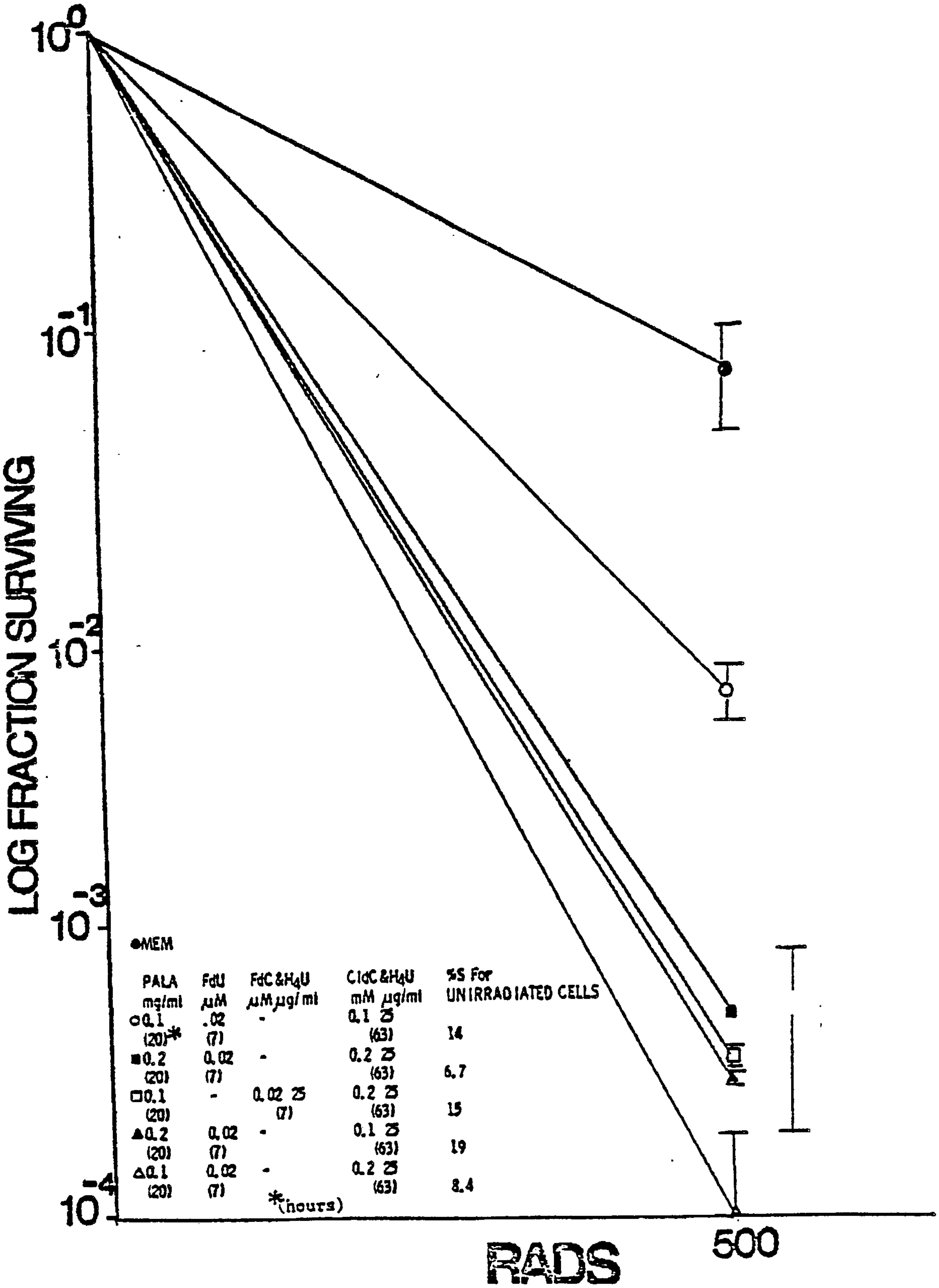

LOG FRACTION SURVIVING
10^0
10^-1
10^-2
10^-3
10^-4
RADS
500
●MEM
PALA mg/ml
FdU µM
FdC &H4U µM µg/ml
CldC &H4U mM µg/ml
%S For UNIRRADIATED CELLS
○0.1 (20)* .02 (7) - 0.1 25 (63) 14
■0.2 (20) 0.02 (7) - 0.2 25 (63) 6.7
□0.1 (20) - 0.02 25 (7) 0.2 25 (63) 15
▲0.2 (20) 0.02 (7) - 0.1 25 (63) 19
△0.1 (20) 0.02 (7) - 0.2 25 (63) 8.4
*(hours)


FIG. 7